# EUROPEAN PATENT APPLICATION

(11) **EP 2 130 585 A1**
(43) Date of publication of application: **09.12.2009**
(21) Application number: 08157768.6
(22) Date of filing: 06.06.2008
(51) Int. Cl.: B01J 29/40, C01B 39/36, C10G 11/05, C01B 39/40

(54) **Process for making crystalline metallosilicates**

(71) Applicant: Total Petrochemicals Research Feluy, 7181 Seneffe (Feluy) (BE)
(72) Inventor: Bulut, Metin, B-3001, Heverlee (Leuven) (BE); Jacobs, Pierre, B-1686, Gooik (BE); Minoux, Delphine, B-7181, Familleureux (BE); Nesterenko, Nikolai, B-1400, Nivelles (BE); Dath, Jean-Pierre, 7970, BELOEIL HAINAULT (BE); Van Donk, Sander, B-1180, Uccle (BE)
(74) Representative: Leyder, Francis

(57) **Abstract**

The present invention relates to a process for making a crystalline metallosilicate composition and comprising crystallites having an inner part (the core) and an outer part (the outer layer or shell) such that :
the ratio Si/Metal is higher in the outer part than in the inner part,
the crystallites have a continuous distribution of metal and silicon over the crystalline cross-section,
said process comprising :
a) providing an aqueous medium comprising OH⁻ anions and a metal source,
b) providing an aqueous medium comprising an inorganic source of silicon and optionally a templating agent,
c) optionally providing a non aqueous liquid medium comprising optionally an organic source of silica,
d) mixing the medium a), b) and the optional c) at conditions effective to partly crystallize the desired metallosilicate,
e) cooling down the reaction mixture a)+b)+c) to about room temperature,
f) decreasing the pH of the reaction mixture by at least 0.1 preferably by 0.3 to 4, more preferably by 0.5 to 3,
g) subjecting the resulting mixture of step f) at conditions effective to continue the crystallization of the desired metallosilicate,
h) recovering the desired metallosilicate,

wherein at step d) in the mixture a)+b)+c), before crystallization,
the ratio Si org/Si inorganic is < 0.3 , advantageously < 0.2 and preferably 0,
the molar ratio OH⁻/SiO₂ is at least 0.3, preferably from 0.3 to 0.6 and more preferably from 0.33 to 0.6 .

## Description

### [Field of the invention]

The present invention relates to a process for making crystalline metallosilicates (or zeolites). Zeolites have been demonstrated to possess catalytic properties for various types of hydrocarbon conversions. In addition, the zeolites have been used as adsorbents and catalyst carriers for various types of hydrocarbon conversion processes, and other applications. More precisely the crystalline metallosilicates made by the process of the present invention comprise crystallites having on the outer surface, and close to the outer surface, a ratio of silicon to metal higher than in the inner part of the crystallite. In the following description the outer surface and the part close to the outer surface can be referred as the outer layer or the shell, the inner part can be referred as the core.

### [Background of the invention]

Crystalline metallosilicates are ordered, porous, crystalline material having a definite crystalline structure as determined by x-ray diffraction, possessing a large number of smaller cavities that may be interconnected by pores. The dimensions of these channels or pores are such as to allow adsorption of molecules with certain dimensions while rejecting those with larger dimensions. The interstitial spaces or channels formed by the crystalline network enable zeolites to be used as molecular sieves in separation processes and catalysts and catalyst supports in a wide variety of hydrocarbon conversion processes. Zeolites or metallosilicates are comprised of a lattice of silicon oxide and optionally a metal oxide combined optionally with exchangeable cations such as alkali or alkaline earth metal ions. Although the term "zeolites" includes materials containing silica and optionally alumina, it is recognized that the silica and alumina portions may be replaced in whole or in part with other oxides. For example, germanium oxide can replace the silica portion. The metal cations other than silicon in the oxide framework of metallosilicates may be iron, aluminium, titanium, gallium and boron. Accordingly, the term "Zeolites" means here microporous crystalline metallosilicates materials. The catalytic properties of metallosilicates are the result of the presence of elements different than silicon in the framework of the zeolite. Substitution of metal cations for silicon in the oxide framework gives rise to potential catalytic active sites. The best known metallosilicates are aluminosilicates that exhibit acidic groups in the pores of the crystals. The substitution of silica with elements such as alumina with a lower valence state creates a positive charge deficiency, which can be compensated by a cation such as a hydrogen ion. The acidity of the zeolite can be on the surface of the zeolite and also within the channels of the zeolite. Within a pore of the zeolite, hydrocarbon conversion reactions such as paraffin isomerization, olefin skeletal or double bond isomerization, oligomerisation, disproportionation, alkylation, and transalkylation of aromatics may be governed by constraints imposed by the channel size of the molecular sieve. The acidic protons, present in the interior of the pores, are subject to shape selective constraints. The principles of "shape selective" catalysis have been extensively reviewed, e.g. by N.Y. Chen, W.E. Garwood and F.G. Dwyer in "Shape selective catalysis in industrial applications", 36, Marcel Dekker, Inc., 1989. However, acidic groups can also be present at the external surface of the metallosilicate crystals. These acidic groups are not subject to the shape selective constraints imposed by the crystalline pore-structure. The acidic groups on the external surface is called here external surface acidity. The external surface acidity may catalyse undesirable reactions that decrease the product selectivity. Typical unselective surface catalysed reactions that are not subject to the constraints imposed by the crystalline pore-structure are: (1) extensive oligo/polymerisation of olefins, (2) isomerisation of alkylaromatics, selectively produced inside the constrained pore-structure (3) formation of polycyclic aromatics (4) multiple alkylation of aromatics (5) multiple branching of olefins and/or paraffins and (6) formation of macromolecular type precursors of coke leading to undesired carbon lay down. The relative amount of external surface acidity is determined by the crystal size; small crystals possess more external surface acidity than large crystals. It is often advantageous to reduce the presence of the external surface acidity of the zeolites or metallosilicate in order to improve their process performance. Performance measures include product selectivity, product quality and catalyst stability.

Many prior arts have described crystallites having on the outer surface, and close to the outer surface, a ratio of silicon to metal higher than in the inner part of the crystallite. Said prior arts describe a first type of process wherein a crystallite is produced and then said crystallite is coated by silica or a composition rich in silica. In a second type of process a crystallite is produced and is further treated to remove a part of the metal from the surface layer to obtain a ratio of silicon to metal higher than in the inner part of the crystallite. In a third type of process a crystallite is produced and is further treated to hinder the metal sites in the outer layer. These prior arts are cited in the introduction part of EP 1661859 A1.

Each of EP 1661859 A1 and WO 2006 092657 describes a process to make directly crystallites having on the outer surface, and close to the outer surface, a ratio of silicon to metal higher than in the inner part of the crystallite.
EP 1661859 A1 describes a crystalline metallosilicate composition comprising crystallites having a crystal outer surface layer having a depth of about 10 nm below the outer surface, and an inner part extending inwardly from a depth of about 50 nm below the outer surface, wherein the atomic ratio of silicon to metal in the metallosilicate composition is at least 1.5 times higher in the crystal outer surface layer as compared to that in the inner part. The process for producing said crystalline metallosilicate composition comprises the steps of :
(a) providing a two-phase liquid medium comprising an aqueous liquid phase and a non-aqueous liquid phase, the two-phase liquid medium further comprising at least one silicon-containing compound and at least one metal-containing compound; and
(b) crystallising the crystalline metallosilicate composition from the two-phase liquid medium.
WO 2006 092657 describes a crystalline metallosilicate composition comprising crystallites having a crystal outer surface layer having a depth of about 10 nm below the outer surface, and an inner part extending inwardly from a depth of about 50 nm below the outer surface, wherein the atomic ratio of silicon to metal in the metallosilicate composition is at least 1.75 times higher in the crystal outer surface layer as compared to that in the inner part. The process for producing said crystalline metallosilicate composition comprises the steps of :
(a) providing an aqueous liquid phase comprising at least one silicon-containing compound and at least one metal-containing compound; and
(b) crystallising the crystalline metallosilicate composition from the aqueous liquid phase, the crystallising step having a first stage followed by a second stage and wherein the concentration of the at least one silicon-containing compound in the aqueous liquid phase is increased in the second stage.

It has now been discovered a new process to make said crystallites having on the outer surface, and close to the outer surface, a ratio of silicon to metal higher than in the inner part of the crystallite which is more efficient and more simple to carry out.

### [Brief summary of the invention]

The present invention relates to a process for making a crystalline metallosilicate composition and comprising crystallites having an inner part (the core) and an outer part (the outer layer or shell) such that :
the ratio Si/Metal is higher in the outer part than in the inner part,
the crystallites have a continuous distribution of metal and silicon over the crystalline cross-section,
said process comprising :
   a) providing an aqueous medium comprising OH⁻ anions and a metal source,
   b) providing an aqueous medium comprising an inorganic source of silicon and optionally a templating agent,
   c) optionally providing a non aqueous liquid medium comprising optionally an organic source of silica,
   d) mixing the medium a), b) and the optional c) at conditions effective to partly crystallize the desired metallosilicate,
   e) cooling down the reaction mixture a)+b)+c) to about room temperature,
   f) decreasing the pH of the reaction mixture by at least 0.1 preferably by 0.3 to 4, more preferably by 0.5 to 3,
   g) subjecting the resulting mixture of step f) at conditions effective to continue the crystallization of the desired metallosilicate,
   h) recovering the desired metallosilicate,
wherein at step d) in the mixture a)+b)+c), before crystallization,
the ratio Si org/Si inorganic is < 0.3 , advantageously < 0.2 and preferably 0,
the molar ratio OH⁻/SiO₂ is at least 0.3, preferably from 0.3 to 0.6 and more preferably from 0.33 to 0.6 .

This process provides a solution to enhance the yield of the zeolite crystals in the synthesis.

As a result, the metallosilicates have reduced surface activity relative to the internal pores, which are subject to shape-selective constraints of the pore-structure.

Advantageously the pH of the mixture a)+b)+c), before crystallization, is higher than 13, preferably higher than 13.1, more preferably higher than 13.2, still more preferably higher than 13.3 and most preferred higher than 13.4.

The operating parameters of the crystallization of step g) could be the same or different as the operating parameters in the crystallisation of step d).

Advantageously, the inorganic source of silicon is selected from at least one of precipitated silica, pyrogenic (fumed) silica, and an aqueous colloidal suspension of silica. Preferably, the inorganic source of silicon has a limited solubility in the water before addition of alkali medium.

Preferably, the organic source of silicon is a tetraalkyl orthosilicate.

The decrease of pH in step f) can be made by any means and advantageously by adding a pH lowering agent. Said pH lowering agent can be any water soluble compound or a mixture of compounds used in concentration sufficient for providing the pH of the aqueous solution lower by 0.1 than pH value of reaction mixture after the first crystallization step. The pH is lowered by at least 0.1 preferably by 0.3 to 4, more preferably by 0.5 to 3. Advantageously, the lowering pH agent is an organic or inorganic acids or a salt providing acidic pH. The organic acids may comprise an organic acid such as citric acid, formic acid, oxalic acid, tartaric acid, malonic acid, succinic acid, glutaric acid, adipic acid, maleic acid, phthalic acid, isophthalic acid, fumaric acid, nitrilotriacetic acid, hydroxyethylenediaminetriacetic acid, ethylenediaminetetracetic acid, trichloroacetic acid trifluoroacetic acid or a salt of such an acid (*e*.*g*. the sodium salt) or a mixture of two or more of such acids or salts. The inorganic acids may comprise an inorganic acid such as nitric acid, hydrochloric acid, methansulfuric acid, sulfuric acid, phosphoric, carbonic or a salt of such an acid (*e*.*g*. the sodium or ammonium salts) or a mixture of two or more of such acids or salts.

Advantageously, the metal source is selected from at least one of the metal oxide, a metal salt, and a metal alkoxide.

Advantageously, the metallosilicate is an aluminosilicate (the Metal is Al), and the source of aluminum is advantageously selected from at least one of hydrated alumina dissolved in an alkaline solution, aluminum metal, a water-soluble aluminum salt, such as aluminum sulphate or aluminium nitrate or aluminium chloride, sodium aluminate and an alkoxide, such as aluminum isopropoxide.

Advantageously, the metallosilicate is a borosilicate, and the source of boron is selected from at least one of hydrated boron oxide dissolved in an alkaline solution, a water-soluble boron salt, such as boron chloride, and an alkoxide.

Advantageously, the metallosilicate is a ferrosilicate, and the source of iron is a water soluble iron salt.

Advantageously, the metallosilicate is a gallosilicate, and the source of gallium is a water soluble gallium salt.

Advantageously, the metallosilicate is a titanosilicate, and the source of titanium is selected from at least one of titanium halides, titanium oxyhalides, titanium sulphates and titanium alkoxides.

Advantageously, the non-aqueous liquid medium comprises an organic solvent which is substantially water insoluble or water immiscible. Preferably, the organic solvent comprises at least one of an alcohol having at least 5 carbon atoms or a mercaptan having at least 5 carbon atoms. Preferably, the alcohol has up to 18 carbon atoms and the mercaptan has up to 18 carbon atoms.

Advantageously, the source of OH⁻ anions is sodium hydroxide.

The present invention also provides a crystalline metallosilicate composition comprising crystallites having a crystal outer surface layer having a depth of about 10 nm below the outer surface, and an inner part extending inwardly from a depth of about 50 nm below the outer surface, wherein the atomic ratio of silicon to metal in the metallosilicate composition is advantageously at least 1.5 times higher in the crystal outer surface layer as compared to that in the inner part. The atomic ratio of silicon to metal in the metallosilicate composition is preferably from 1.5 to 15, more preferably from 2 to 10, most preferably from 3 to 5 times higher in the crystal outer surface layer as compared to that in the inner part. Preferably, the inner part has a silicon/metal atomic ratio of from 11 to 1000, more preferably from 22 to 500, and the crystal surface has a silicon/metal atomic ratio of from 17 to 15000, more preferably from 34 to 5000. Preferably, the inner part has a substantially constant silicon/metal atomic ratio.

The present invention additionally provides the use of the crystalline metallosilicate composition obtained by the process of the present invention as a catalyst component in a hydrocarbon conversion process.

Advantageously, the medium b) and c) are mixed first and medium a) is further added slowly in the mixture b)+c) until a hydrogel is obtained. Then the crystallization is made by heating advantageously under stirring conditions. Further to the crystallization there is a cooling, a filtration, a washing, a drying and finally a calcination step as in any zeolite synthesis.

### [Detailed description of the invention]

Metallosilicates characterised by a spatial distribution of the constituting elements and characterised by a surface enriched in silicon that can be produced by the process of the present invention can be any of the synthetic crystalline zeolites able to be synthesized in basic medium.
Advantageously, the zeolite according to invention is selected from the group MFI (ZSM-5, silicalite, TS-1), MEL (ZSM-11, silicalite-2, TS-2), MTT (ZSM-23, EU-13, ISI-4, KZ-1), MFS (ZSM-57), HEU (Clinoptilolite), FER (ZSM-35, Ferrierite, FU-9, ISI-6, NU-23, Sr-D), TON (ZSM-22, Theta-1, ISI-1, KZ-2 and NU-10), LTL (L), MAZ (mazzite, Omega, ZSM-4). These zeolites and their isotypes are described in "Atlas of Zeolite Structure Types", eds. W. H. Meier, D. H. Olson and Ch. Baerlocher, Elsevier, Fourth Edition, 1996, which is hereby incorporated by reference. The structure types are provided by the "IUPAC Commission of Zeolite Nomenclature". The conventional procedure for the synthesis of these zeolite is given in "Verified synthesis of zeolytic materials, eds H. Robson, Elsevier 2001.

The metallosilicates obtained by the process of the present invention may comprise a charge balancing cation M selected from the group consisting of hydrogen, ammonium, monovalent, divalent and trivalent cations and mixtures thereof.

The sources of the various elements of the metallosilicate may be any of those found in the commerce or prepared on purpose. For example, the source of silicon may be a silicate, e.g., a tetraalkyl orthosilicate, precipitated or pyrogenic silica, or preferably an aqueous colloidal suspension of silica. When the metallosilicate is an aluminosilicate zeolite, the source of aluminum is preferably hydrated alumina dissolved in an alkaline solution or aluminum metal, a water-soluble aluminum salt, e.g., aluminum sulphate or aluminium chloride, sodium-aluminate or an alkoxide, e.g., aluminum isopropoxide. When the metallosilicate is a borosilicate zeolite, the source of boron is preferably hydrated boron oxide dissolved in an alkaline solution or a water-soluble boron salt, e.g., boron chloride or an alkoxide. When the metallosilicate is a ferrosilicate or gallosilicate, the source of iron or gallium can almost be any iron or gallium salts that is readily soluble in water. When the metallosilicate is titanosilicate, the source of titanium can be titanium halides, titanium oxyhalides, titanium sulphates or titanium alkoxides. The atomic ratio of silicon to metal depends on the metal and on the use of the metallosilicate and is at least 2/1 to about 10000/1, preferably from 5/1 to about 5000/1 and most preferred from about 10/1 to 1000/1. Optionally one or more templating agent (or directing agent), such as organic or inorganic compounds containing nitrogen, oxygen, sulfur, or phosphorous may be introduced into the synthesis mixture. When the directing agent is a cation, it may also be introduced in the form of a mixture of hydroxide and salt, e.g., a halide. The agent used will depend on the metallosilicate prepared by the process. The amount of the directing agent depends on the metallosilicate prepared by the process. The source of M cations may be alkali or alkaline earth hydroxides or salts. M may also be ammonium hydroxide or salts. Together with the directing agent(s) the M cation will impact the pH of the crystallising medium. The proportion of the source of OH⁻ in the aqueous medium a) has to be in accordance with the templating agent and the M cation to comply with the molar ratio OH⁻/SiO₂ of at least 0.3 and preferably from 0.3 to 0.6 in the mixture a)+b)+c) at step d).

The organic solvent medium preferably is essentially water-insoluble or water-immiscible. The organic solvent medium preferably contains at least one alcohol or mercaptan, which is essentially water-insoluble. Examples of alcohols or mercaptans which are essentially water-insoluble are alcohols or mercaptans with at least 5 up to about 18 carbons. The organic solvent medium can optionally contain other water-insoluble organic compounds that do not bear an alcohol or mercaptan functional group. A person skilled in the art knows how to alter the hydrophobicity of the organic medium when required for the synthesis of a particular metallosilicate. Organic compounds that may be employed together with the required amount of water-insoluble alcohols or mercaptans can be halohydrocarbons, paraffinic, cycloparaffinic, aromatic hydrocarbons or mixture thereof.

The order of mixing of a), b) and c) is not essential and will depend on the zeolite being prepared. Optionally the crystallisation medium (a)+b)+c)) may be aged at a temperature at which no crystallisation occurs, optionally nucleation may be started. Persons skilled in the art know equipment used to prepare the zeolite crystals of the present invention. Generally, metallosilicates can be prepared by using autoclaves, which have sufficient agitation to homogenise the crystallisation mixture during heat up until the effective nucleation and crystallisation temperature of the mixture is achieved. The crystallisation vessel can be made of a metal or metal alloys resisting the conditions of the crystallisation or optionally can be coated with a fluorocarbon such as Teflon®™. Other means of introducing agitation known to one skilled in the art can be employed, such as pumping the synthesis mixture from one part of the autoclave to another.

In an advantageous embodiment the crystallisation medium obtained by mixing a), b) and c) is maintained under stirring conditions at room temperature for a period of 10 minutes to 2 hours. Then the crystallization medium is submitted to autogenous pressure and elevated temperature. The reaction mixture is heated up to the crystallization temperature that may range from about 120°C to 250°C, preferably from 130°C to 230°C, most preferably from 160°C to 220°C. Heating up to the crystallization temperature is typically carried for a period of time ranging from about 0,5 to about 30 hours, preferably from about 1 to 12 hours, most preferably from about 2 to 9 hours. The temperature may be increased stepwise or continuously. However, continuous heating is preferred. The crystallization medium may be kept static or agitated by means of tumbling or stirring the reaction vessel during hydrothermal treatment. Preferably, the reaction mixture is tumbled or stirred, most preferably stirred. The temperature is then maintained at the crystallization temperature for a period of time ranging from 2 to 200 hours. Heat and agitation is applied for a period of time effective to form crystalline product. In a specific embodiment, the reaction mixture is kept at the crystallization temperature for a period of from 16 to 96 hours. Any oven such as a conventional oven and a microwave oven can be used.
The operating parameters of the crystallization of step g) could be the same or different as the operating parameters in the crystallisation of step d).

Typically, the crystalline metallosilicate is formed as a slurry and can be recovered by standard means, such as by sedimentation, centrifugation or filtration. The separated crystalline metallosilicate is washed, recovered by sedimentation, centrifugation or filtration and dried at a temperature of typically from about 25°C to about 250°C, and more preferably from 80°C to about 120°C. Calcination of the metallosilicate is known per se. As a result of the metallosilicate crystallization process, the recovered metallosilicate contains within its pores at least a portion of the template used. In a preferred embodiment, activation is performed in such a manner that the template is removed from the metallosilicate, leaving active catalytic sites with the microporous channels of the metallosilicate open for contact with a feedstock. The activation process is typically accomplished by calcining, or essentially heating the metallosilicate comprising the template at a temperature of from 200 to 800°C in the presence of an oxygen-containing gas. In some cases, it may be desirable to heat the metallosilicate in an environment having a low oxygen concentration. This type of process can be used for partial or complete removal of the template from the intracrystalline pore system.
Once the crystalline metallosilicate is made, it can be used as itself as a catalyst. In another embodiment it can be formulated into a catalyst by combining the crystalline metallosilicate with other materials that provide additional hardness or catalytic activity to the finished catalyst product.

The crystals prepared by the instant invention can be formed into a wide variety of forms. In cases where a catalyst is produced from the metallosilicate produced by the present invention, the catalyst needs to possess a shape to be applicable in industrial reactors. The crystals can be shaped before drying or partially dried and then shaped or the crystals can be calcined to remove organic template and then shaped. In the case of many catalysts, it is desirable that crystalline zeolites prepared by the process of the present invention are incorporated with binder material resistant to the temperature and other conditions employed in organic conversion processes. It will be easily understood by the person skilled in the art that binder material does not contain the metal element that is incorporated into the framework of the metallosilicate characterised by a spatial distribution of the constituting elements and characterised by a surface enriched in silicon. In addition, the binder material does not contain elements that destroy the spatial distribution of the constituting elements of the metallosilicate or the surface enriched in silicon of the metallosilicate. Examples of binder material may be composited with a porous matrix material, such as silica, zirconia, magnesia, titania, silica-magnesia, silica-zirconia, silica-thoria, and silica-titania, as well as ternary compositions, such as silica-magnesia-zirconia. The relative proportions of metallosilicate component and binder material will vary widely with the metallosilicate content ranging from between about 1 to about 99 percent by weight, more preferably in the range of about 10 to about 85 percent by weight of metallosilicate component, and still more preferred from about 20 to about 80 percent. The metallosilicate prepared by the process of the present invention may be further ion exchanged after calcination to remove organic template as is known in the art either to replace at least in part the original charge-balancing cations present in the metallosilicate with a different cation, e.g. a Group IB to VIII of the Periodic Table metal such as tungsten, molybdenum, nickel, copper, zinc, palladium, platinum, calcium or rare earth metal, or to provide a more acidic form of the zeolite by exchange of original charge-balancing cation with ammonium cations, followed by calcination of the ammonium form to provide the acidic hydrogen form. The acidic form may be readily prepared by ion exchange using a suitable reagent such as ammonium nitrate, ammonium carbonate or protonic acids, like HCI, HNO3 and H3PO4. The metallosilicate may then be calcined at a temperature of 400 to 550° C to remove ammonia and create the hydrogen form. Particularly preferred cations will depend on the use of the metallosilicate and include hydrogen, rare earth metals, and metals of Groups IIA, 1IIA, IVA, IB, IIB, IIIB, IVB, and VIII of the Periodic Table of the Elements. The metallosilicate prepared by the process of the present invention may be further supported by at least one different precursor of metals that have catalytic activity after known pretreatments, e.g. a Group IIA, IIIA to VIIIA, IB, IIB, IIIB to VIB of the Periodic Table metal such as tungsten, molybdenum, nickel, copper, zinc, palladium, platinum, gallium, tin, and/or tellurium metal precursors.

Since the metallosilicate of the present invention characterised by a spatial distribution of the constituted elements and characterised by a surface enriched in silicon have controlled catalytic activity which is the result of the presence of catalytic active sites mainly in the inner part of the metallosilicate crystals and largely the absence of unselective catalytic active sites near the external surface of the metallosilicate crystals, which can cause undesirable side reactions to occur, the metallosilicate of the present invention by itself or in combination with one or more catalytically active substances can have high activity, high selectivity, high stability, or combinations thereof when used as catalysts for a variety of hydrocarbon conversion processes. Examples of such processes include, as non-limiting examples, the following:
1. The alkylation of aromatic hydrocarbons with light olefins to provide short chain alkyl aromatic compounds, e.g., the alkylation of benzene with propylene to provide cumene and alkylation of benzene with ethylene to provide ethylbenzene. Typical reaction conditions include a temperature of from about 100° C. to about 450° C, a pressure of from about 5 to about 80 bars, and an aromatic hydrocarbon weight hourly space velocity of from 1 hr⁻¹ to about 100 hr⁻¹.
2. The alkylation of polycyclic aromatic hydrocarbons with light olefins to provide short chain alkyl polycyclic aromatic compounds, e.g., the alkylation of naphthalene with propylene to provide mono-or di-isopropyl-naphthalene. Typical reaction conditions include a temperature of from about 100° C to about 400° C, a pressure of from about 2 to about 80 bars, and an aromatic hydrocarbon weight hourly space velocity of from 1 hr⁻¹ to about 100 hr⁻¹
3. The alkylation of aromatic hydrocarbons, e.g., benzene and alkylbenzenes, in the presence of an alkylating agent, e.g., alkyl halides and alcohols having 1 to about 20 carbon atoms. Typical reaction conditions include a temperature of from about 100° C to about 550° C, a pressure of from about atmospheric to about 50 bars, a weight hourly space velocity of from about 1 hr⁻¹ to about 1000 hr⁻¹ and an aromatic hydrocarbon/alkylating agent mole ratio of from about 1/1 to about 20/1.
4. The alkylation of aromatic hydrocarbons, e.g., benzene, with long chain olefins, e.g., C14 olefin. Typical reaction conditions include a temperature of from about 50° C to about 300° C, a pressure of from about atmospheric to about 200 bars, a weight hourly space velocity of from about 2 hr⁻¹ to about 1000 hr⁻¹ and an aromatic hydrocarbon/olefin mole ratio of from about 1/1 to about 20/1.
5. The alkylation of phenols with olefins or equivalent alcohols to provide long chain alkyl phenols. Typical reaction conditions include temperatures from about 100° C to about 250° C, pressures from about I to 50 bars and total weight hourly space velocity of from about 2 hr⁻¹ to about 10 hr⁻¹.
6. The transalkylation of aromatic hydrocarbons in the presence of polyalkylaromatic hydrocarbons. Typical reaction conditions include a temperature of from about 150° C to about 550° C, a pressure of from about atmospheric to about 100 bars, a weight hourly space velocity of from about 1 hr⁻¹ to about 500 hr⁻¹ and an aromatic hydrocarbon/polyalkylaromatic hydrocarbon mole ratio of from about 1/1 to about 20/1.
7. The isomerization of aromatic (e.g., xylene) feedstock components. Typical reaction conditions for such include a temperature of from about 200° C to about 550° C, a pressure of from about 1 bars to about 50 bars, a weight hourly space velocity of from about 0.1 hr⁻¹ to about 200 hr⁻¹ and a hydrogen/hydrocarbon mole ratio of from about 0 to about 100.
8. The disproportionation of toluene to make benzene and paraxylene. Typical reaction conditions including a temperature of from about 200° C to about 600° C, a pressure of from about atmospheric to about 60 bar, and a weight hourly space velocity of from about 0.1 hr⁻¹ to about 30 hr⁻¹.
9. The catalytic cracking of naphtha feed to produce light olefins. Typical reaction conditions include from about 450° C to about 650° C, pressures of atmospheric to about 8 bars and weight hourly space velocity of from about 5 hr⁻¹ to 50 hr⁻¹.
10. The catalytic cracking of butenes feed to produce light olefins, e.g. propylene. Typical reaction conditions include from about 450° C to about 650° C, pressures of atmospheric to about 8 bars and weight hourly space velocity of from about 5 hr⁻¹ to 50 hr⁻¹.
11. The catalytic cracking of high molecular weight hydrocarbons to lower weight hydrocarbons. The metallosilicate of the instant invention may be employed in combination with conventional catalyst used in fluid catalytic cracking units. Typical reaction conditions for catalytic cracking include temperatures of from about 450° C to about 650° C, pressures of from about 0.1 bar to about 10 bars, and weight hourly space velocities of from about 1 hr⁻¹ to about 300 hr⁻¹.
12. The dewaxing of hydrocarbons by selectively removing straight chain paraffins. Typical reaction conditions include a temperature between about 200° C and 450° C, a pressure from 10 to up to 100 bars and a liquid hourly space velocity from 0.1 hr⁻¹ to 20 hr⁻¹.
13. The hydrocracking of heavy petroleum feedstocks. The metallosilicate catalyst contains an effective amount of at least one hydrogenation component of the type employed in hydrocracking catalysts.
14. A combination hydrocracking/dewaxing process in which optionally more than one metallosilicate or combinations of metallosilicate with other zeolites or molecular sieves are employed.
15. The conversion of light paraffins to olefins and/or aromatics. Typical reaction conditions include temperatures from about 425° C to about 750° C and pressures from about I to about 60 bars.
16. The conversion of light olefins to gasoline, distillate and lube range hydrocarbons. Typical reaction conditions include temperatures of from about 175° C to about 450° C and a pressure of from about 3 to about 100 bars.
17. The conversion of naphtha (e.g. C6-C10) into products having a substantial higher octane aromatics content by contacting the hydrocarbon feed with the catalyst at a temperature in the range of from about 400° C to 600° C, preferably 480° C to 550° C at pressures ranging from atmospheric to 40 bar and liquid hourly space velocities ranging from 0.1 hr⁻¹ to 35 hr⁻¹.
18. The reaction of alcohols with olefins to provide mixed ethers, e. g., the reaction of methanol or ethanol with isobutene and/or isopentene to provide methyl-t-butyl ether (MTBE) or ethyl-t-butyl ether (ETBE) and/or t-amyl methyl ether (TAME) or t-amyl-ethyl-ether (TAEE). Typical conversion conditions including temperatures from about 20° C to about 250° C, pressures from 2 to about 100 bar, a liquid hourly space from about 0.1 hr⁻¹ to about 200 hr⁻¹ and an alcohol to olefin molar feed ratio from about 0.2/1 to about 3/1.
19. The decomposition of ethers like MTBE, ETBE, TAME or TAEE into isobutene and isopentenes and the corresponding alcohol. Typical conversion conditions including temperatures from about 20° C to about 300° C, pressures from 0.5 to about 10 bars, a liquid hourly space from about 0.1 hr⁻¹ to about 200 hr⁻¹.
20. The conversion of oxygenates, e.g., alcohols, such as methanol, or ethers, such as dimethylether, or mixtures thereof to hydrocarbons including olefins and aromatics with reaction conditions including a temperature of from about 275° C to about 600° C, a pressure of from about 0.5 bar to about 60 bar and a liquid hourly space velocity of from about 0.1 hr⁻¹ to about 100 hr⁻¹
21. The oligomerization of straight and branched chain olefins having from about 2 to about 10 carbon atoms. The oligomers that are the products of the process have 6 to about 50 carbons, which are useful for both fuels blending feedstock, as solvents, lube oils, alkylation agents and reactants for preparing various kinds of oxygen containing chemicals. The oligomerization process is generally carried out at a temperature in the range of from about 150° C to about 350° C, a liquid hourly space velocity of from about 0.2 hr⁻¹ to about 70 hr⁻¹ and a pressure of from about 5 to about 100 bar.

The invention is illustrated by the following non-limiting Examples.
In the following Examples, the techniques used to produce and characterise the obtained materials are given.
X-ray diffraction was used to obtain a diffraction pattern, to ensure that desired crystal structure is confirmed or to detect presence of foreign crystalline phases and to determine degree of crystallinity compared with a reference zeolite. The diffractometer was a Philips PW1830 (Co Kα). The spatial distribution of the constituting elements was measured by means of "secondary ion mass spectrometry" or SIMS. The apparatus used was a CAMECA TOF-SIMS IV. To avoid charge effects, zeolites being non-conductive materials, a low energy electron floodgun was used. To realise in depth composition profiles, a sputtering gun was used simultaneously to the analysis gun. Both guns used argon as primary ions, the energy of the sputtering gun ion beam being 3 keV for a current density of 20 nA, and the analysis gun having an energy of 10 keV with a current of 1 pA.
The sputtering gun eroded a surface area of 200 x 200 micron, and the surface analysis gun scanned a surface area of about 5 x 5 micron. Profiles were performed in non-interlaced mode, meaning that analysis and sputtering of the samples was completely dissociated. The cycle sequence was as follows: 30 seconds analysis-30 seconds sputtering-2 seconds pausing. Zeolite powder was compacted and pressed into a wafer. The wafers were fixed on a support and placed in a vacuum of 10⁻⁶ to 10⁻⁷ Torr. After degassing for a period of 24 hours analysis was performed. Only monoatomic species of aluminium and silicon were taken into account for concentration profiles and only the double charged cations are considered for quantitative measurements (Si²⁺/Al²⁺) . A prior calibration had been realised on zeolites with well known Si/Al ratios. Under the circumstances of the analysis the calibration curve responded to the following equation:
Si/Al in framework = 2.1008 Si²⁺/Al²⁺ by SIMS
By means of a profilometer the erosion velocity had been measured and corresponded to 0.17 nm/second.

An MFI aluminosilicate was prepared by mixing solutions a), b) and c).
***Solution a) :*** xxx g of sodium hydroxyde in xxx ml of distilled water and xxx g of AI(NO₃)₃.9 H₂O was mixed, followed by 15 min homogenisation (Table 2)
***Solution b) :*** xxx g of template in xxx ml of distilled water and xxx ml of colloidal silica solution containing 40%wt SiO₂ (Ludox AS-40) (Table 2).
***Solution c) :*** xxx ml of hexanol-1 (Table 2)

Solution b) and c) were mixed in an autoclave for a period of 15 minutes and a hydrogel was obtained by adding slowly solution a). After stirring for 30 min at room temperature, the crystallization reaction was performed.

In case of the examples 1-3, the first crystallization was performed via 3 hours microwave thermal treatment under autogeneous pressure at 170 °C (stirred with magnetic bar). Then the sample 1 was directly recovered from reaction medium after the first synthesis.
The reaction media of sample 2 and 3 are cooled down till room temperature and 1 M H₂SO₄ solution is added to decrease the pH by 2 (table 2, 3). The second synthesis was performed under autogeneous pressure via 16 hours microwave thermal treatment at 170 °C.

In case of the examples 4-8, the first crystallization was performed via 18 hours microwave thermal treatment under autogeneous pressure at 170 °C (stirred with magnetic bar). Then the sample 4 was directly recovered from reaction medium after the first synthesis.
The reaction media of sample 5-7 are cooled down till room temperature and 1 M H₂SO₄ solution is added to decrease the pH by 2 (table 2, 3). The second synthesis was performed under autogeneous pressure via 21 hours microwave thermal treatment at 170 °C.
The reaction media of sample 8 is cooled down till room temperature and a buffer solution containing 0.05 M Na₂CO₃/NaHCO₃ is added to decrease the pH (table 2, 3). The second synthesis was performed under autogeneous pressure via 21 hours microwave thermal treatment at 170 °C.

The product was then cooled and washed with 0.75 liters of distilled water, dried at 110°C for 16 hours and then calcined at 600°C for 5 hours in order to remove the organic material.

The exact amount of each compound is reported in table 2, and the synthesis conditions and molar ratio of the reagent in table 1. Table 3 shows the pH evolution during the synthesis.

The ratios Si/Al are displayed on
fig 1 (ex 1-3),
fig 2 (ex 4-6),
fig. 3 (ex 7)
fig. 4 (ex 8)
the XRD paterns
fig 5 (ex 1-3)
fig 6 (ex 4-6)
fig. 7 (ex 7)
fig. 8 (ex 8)
The SEM images
fig 9 (ex 1-3)

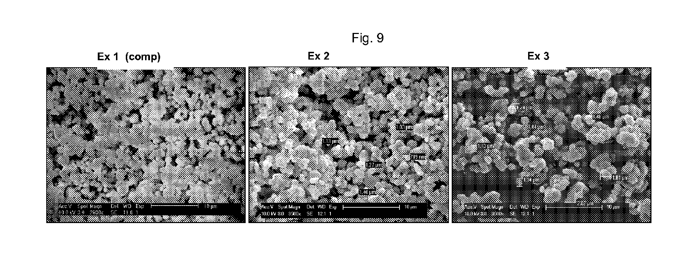

The resulting crystals show no significant difference in morphology and crystal size, when samples 2 and 3 are compared with sample 1 (figure 9).

The powder yield increases from 74.98 (ex 1) % to 90.64 and 93.10 for the samples 2 and 3, and from 73.19 % (ex 4) to 90.32 % and 84.48 % for sample 5 and 6 respectively.

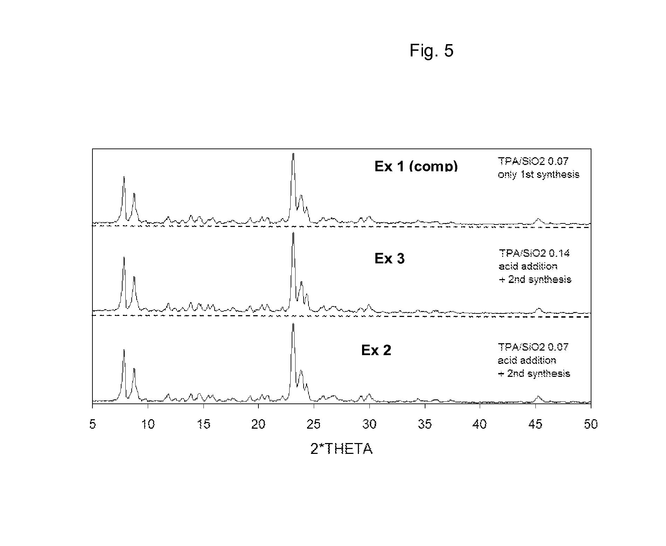

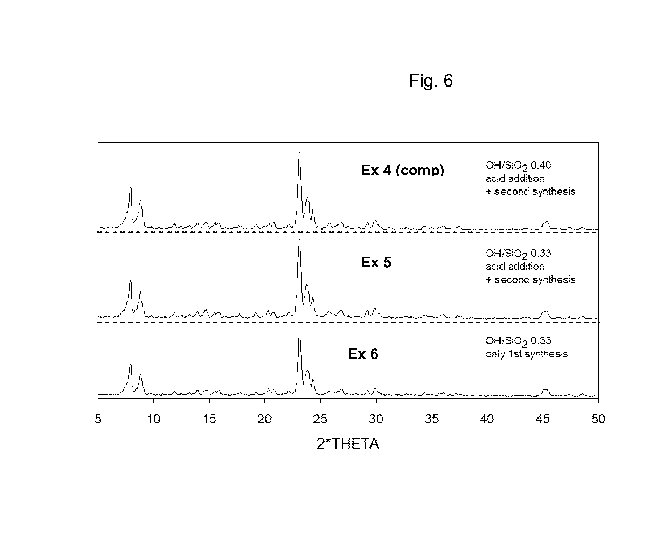

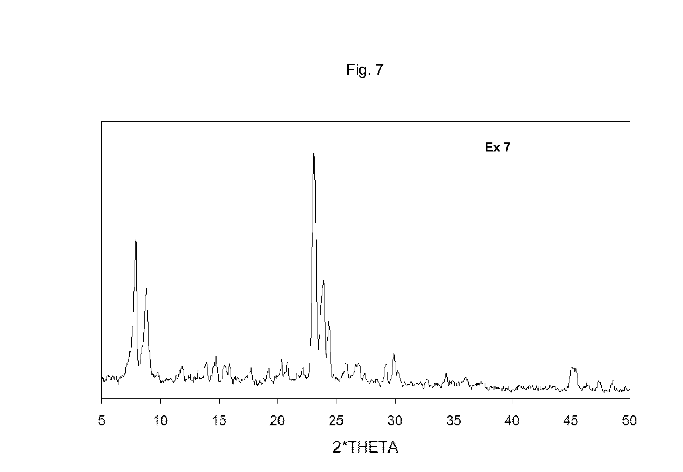

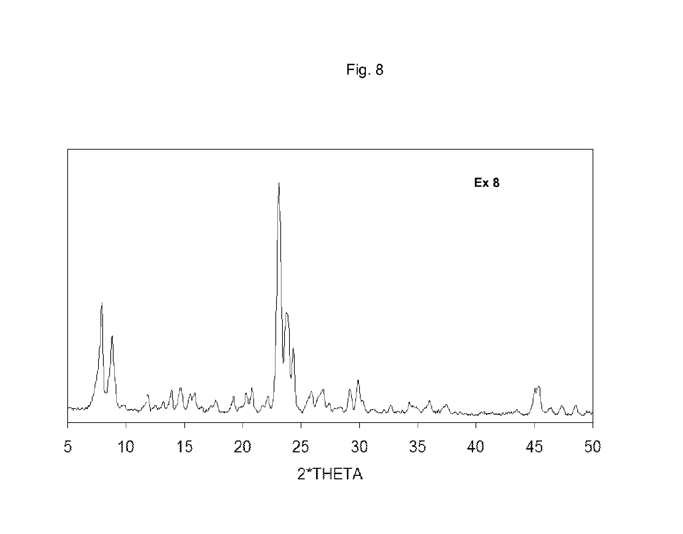

The crystallinity and phase composition also remain similar (figures 5-8).

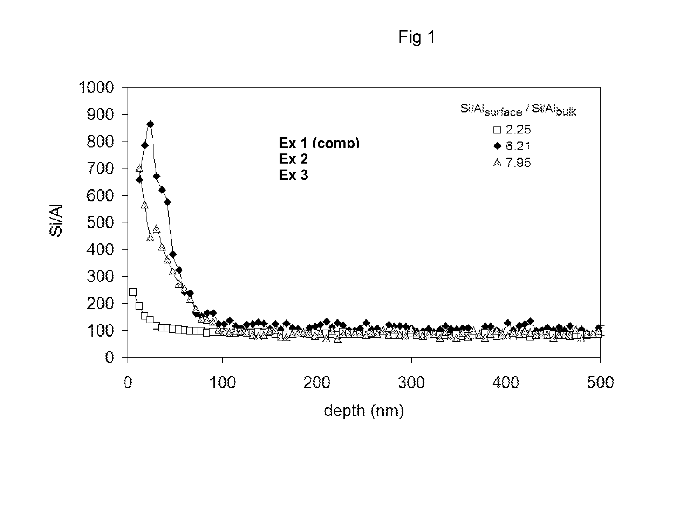

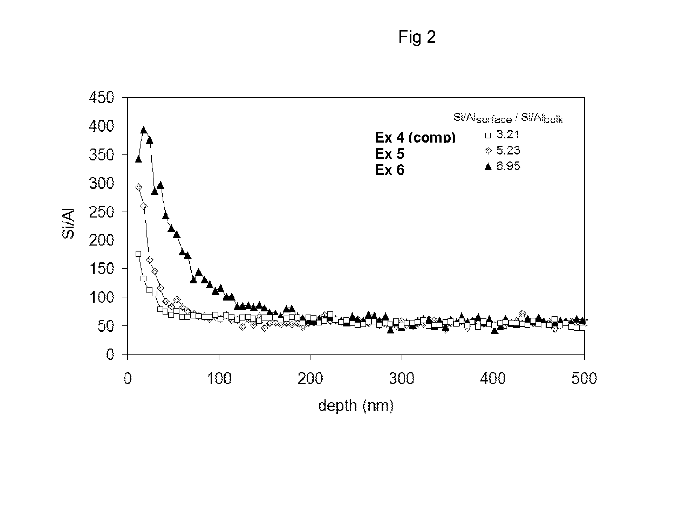

The SIMS profile shows a more expressed gradient in case of the 2-step synthesis, in respect to one-step procedure with an increase of the Si/Al_{surface} / Si/Al_{bulk} ratio (figure 1-2).

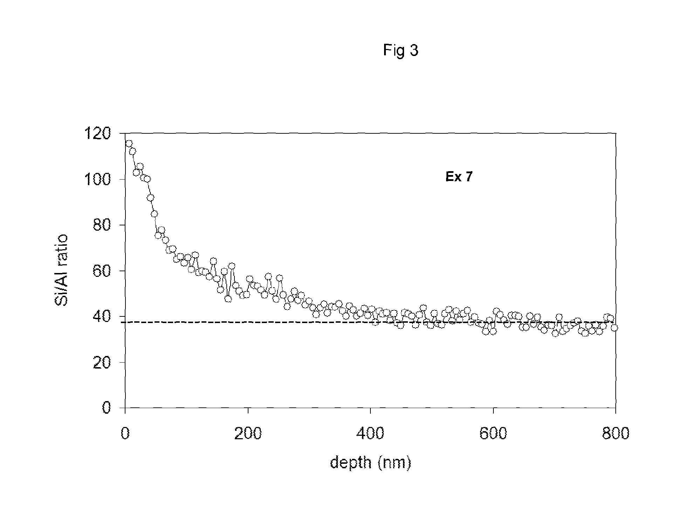

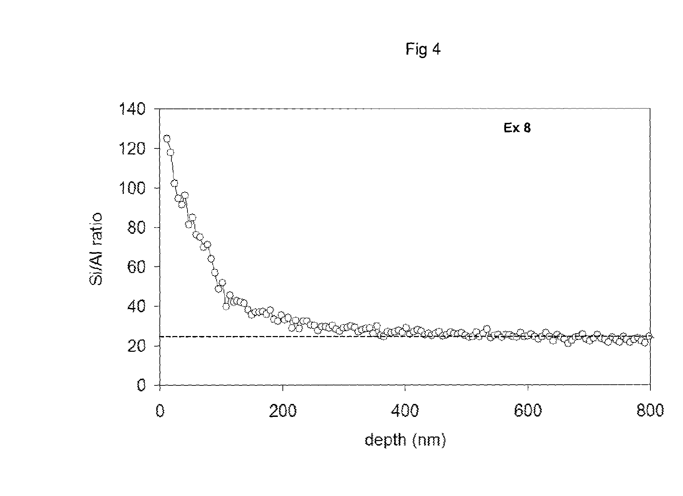

Fig. 3,4 demonstrated that this recipe is applicable and gives good results also for the samples with low Si/Al ratio.

**Table 1**

| | | | molar composition | | | | | volume | |
|---|---|---|---|---|---|---|---|---|---|
| Example | Structure | Template | Si/Al | Template/SiO₂ | Na/SiO₂ | OH⁻/SiO₂ | H₂O/SiO₂ | Vorg/Vaq | Solvent |
| 1 (comp) | MFI | TPA | 115 | 0.07 | 0.33 | 0.33 | 32 | 0.5 | 1-hexanol |
| 2 | MFI | TPA | 115 | 0.07 | 0.33 | 0.33 | 32 | 0.5 | 1-hexanol |
| 3 | MFI | TPA | 115 | 0.14 | 0.33 | 0.33 | 32 | 0.5 | 1-hexanol |
| 4 (comp | MFI | TPA | 70 | 0.07 | 0.33 | 0.33 | 32 | 0.5 | 1-hexanol |
| 5 | MFI | TPA | 70 | 0.07 | 0.33 | 0.33 | 32 | 0.5 | 1-hexanol |
| 6 | MFI | TPA | 70 | 0.07 | 0.4 | 0.4 | 32 | 0.5 | 1-hexanol |
| 7 | MFI | TPA | 40 | 0.07 | 0.4 | 0.4 | 32 | 0 | none |
| 8 | MFI | TPA | 25 | 0.07 | 0.45 | 0.45 | 32 | 0 | none |

| | | | | | | | | | |
|---|---|---|---|---|---|---|---|---|---|
| "TPA" means tetrapropylammonium cation | | | | | | | | | |

**Table 2**

| | Solution a) | | | Solution c) | Solution b) | | | |
|---|---|---|---|---|---|---|---|---|
| Example | NaOH (g) | H₂O (ml) | AI(NO₃)₃×9 H₂O (g) | Hexanol-1 (ml) | Ludox AS-40 (ml) | H2O (ml) | Template (g) | pH lowering agent (ml) |
| 1 (comp) | 0.4583 | 10 | 0.1133 | 10,000 | 4.0219 | 5.9781 | 0.6604 | 0 (1M H2SO4) |
| 2 | 0.4583 | 10 | 0.1133 | 10,000 | 4.0219 | 5.9781 | 0.6604 | 2.5 (1M H2SO4) |
| 3 | 0.4583 | 10 | 0.1133 | 10,000 | 4.0219 | 5.9781 | 1.3207 | 2.75 (1M H2SO4) |
| 4 (comp) | 0.4583 | 10 | 0.1861 | 10,000 | 4.0219 | 5.9781 | 0.6604 | 0 (1M H2SO4) |
| 5 | 0.4583 | 10 | 0.1861 | 10,000 | 4.0219 | 5.9781 | 0.6604 | 2.5 (1M H2SO4) |
| 6 | 0.5556 | 10 | 0.1861 | 10,000 | 4.0219 | 5.9781 | 0.6604 | 3.5 (1M H2SO4) |
| 7 | 0.2778 | 5 | 0.1628 | 0,000 | 2.0109 | 2.9891 | 0.3302 | 1.0 (1M H2SO4) |
| 8 | 0.4688 | 4.4836 | 0.3908 | 0,000 | 3.0164 | 7.5000 | 0.4953 | 3 ml (0.05 M Na₂CO₃) + 5ml(0.05 M NaHCO₃) |

**Table 3**

| | pH evolution | | | | | | | |
|---|---|---|---|---|---|---|---|---|
| Example | 1 | 2 | 3 | 4 | 5 | 6 | 7 | 8 |
| initial | 13.43 | 13.43 | 13.46 | 13.40 | 13.40 | 13.57 | 13.52 | 13.612 |
| After the first crystallization | 12.05 | 12.05 | 12.12 | 12.40 | 12.40 | 12.22 | 11.82 | 12.420 |
| After adding 1M H3PO4 | - | 10.17 | 10.23 | - | 10.38 | 10.48 | 10.03 | 9.650 |
| After second synthesis | - | 11.34 | 11.26 | - | 10.92 | 10.558 | 11.92 | 11.090 |

## Claims

1. Process for making a crystalline metallosilicate composition and comprising crystallites having an inner part (the core) and an outer part (the outer layer or shell) such that :
the ratio Si/Metal is higher in the outer part than in the inner part,
the crystallites have a continuous distribution of metal and silicon over the crystalline cross-section,
said process comprising :
a) providing an aqueous medium comprising OH⁻ anions and a metal source,
b) providing an aqueous medium comprising an inorganic source of silicon and optionally a templating agent,
c) optionally providing a non aqueous liquid medium comprising optionally an organic source of silica,
d) mixing the medium a), b) and the optional c) at conditions effective to partly crystallize the desired metallosilicate,
e) cooling down the reaction mixture a)+b)+c) to about room temperature,
f) decreasing the pH of the reaction mixture by at least 0.1,
g) subjecting the resulting mixture of step f) at conditions effective to continue the crystallization of the desired metallosilicate,
h) recovering the desired metallosilicate,
wherein at step d) in the mixture a)+b)+c), before crystallization,
the ratio Si org/Si inorganic is < 0.3 ,
the molar ratio OH⁻/SiO₂ is at least 0.3.

2. Process according to claim 1 wherein the ratio Si org/Si inorganic is < 0.2.

3. Process according to claim 2 wherein the ratio Si org/Si inorganic is 0.

4. Process according to any one of the preceding claims wherein at step f) the decrease of pH is of 0.3 to 4 .

5. Process according to claim 4 wherein at step f) the decrease of pH is of 0.5 to 3.

6. Process according to any one of the preceding claims wherein at step d) in the mixture a)+b)+c), the molar ratio OH⁻/SiO₂ is from 0.3 to 0.6 .

7. Process according to any one of the preceding claims wherein the metallosiliocate is an aluminosilicate.

8. Process according to any one of the preceding claims wherein the metallosilicate is an MFI.

9. Process according to any one of claims 1 to 7 wherein the metallosilicate is selected from the group MEL, MTT, MFS, HEU, FER, TON, LTL, MAZ.

10. Process according to any one of the preceding claims wherein he atomic ratio of silicon to metal in the metallosilicate composition is from 1.5 to 15 times higher in the crystal outer surface layer as compared to that in the inner part.

11. Process according to claim 10 wherein the atomic ratio of silicon to metal in the metallosilicate composition is from 2 to 10 times higher in the crystal outer surface layer as compared to that in the inner part.

12. Process according to claim 11 wherein the atomic ratio of silicon to metal in the metallosilicate composition is from 3 to 5 times higher in the crystal outer surface layer as compared to that in the inner part.

13. Process according to any one of the preceding claims wherein the inorganic source of silicon is selected from at least one of precipitated silica, pyrogenic silica (or fumed silica), and an aqueous colloidal suspension of silica.

14. Process according to any one of the preceding claims wherein the medium b) and c) are mixed first and medium a) is further added slowly in the mixture b)+c) until a hydrogel is obtained.

15. Use of the crystalline metallosilicate composition obtained by the process according to any one of the preceding claims as a catalyst component in a hydrocarbon conversion process.
